# EUROPEAN PATENT SPECIFICATION

(11) **EP 2 915 505 B1**
(45) Date of publication and mention of the grant of the patent: **10.01.2018**
(21) Application number: 14186582.4
(22) Date of filing: 26.09.2014
(51) Int. Cl.: A61F 2/00

(54) **Support device used in medical breast reconstruction**
Trägervorrichtung für medizinische Brustrekonstruktion
Dispositif de support utilisé dans la reconstruction médicale du sein

(30) Priority: 07.03.2014 US 201461966998 P
(43) Date of publication of application: 09.09.2015
(73) Proprietor: Novus Scientific AB, 754 50 Uppsala (SE)
(72) Inventor: Egnelöv, Per, 754 40 Uppsala (SE)
(74) Representative: Brann AB

(56) References cited:
- EP-A1- 2 556 806
- WO-A1-99/16389
- WO-A2-2011/086537
- WO-A2-2013/012635
- FR-A- 1 085 676
- US-A1- 2010 023 029
- US-A1- 2013 325 119

## Description

### Field of the Invention

The present invention relates to a medical support device for supporting a breast implant or breast tissue, and relates in particular to a medical support device, which comprises a pre-shaped, three-dimensional, generally hemispherical, synthetic and preferably resorbable mesh structure having a number of portions with individual and specific mechanical characteristics.

### Background of the Invention

A medical breast reconstruction is a procedure that typically involves the use of prosthetic breast implants, e.g. silicone or saline implants, which are placed either below or above the breast muscle, to recreate a female breast. In other, also common techniques, the patient's own body tissue can be used to reconstruct a breast. Within the art of reconstructive and cosmetic breast surgery it is further common to at least partly place the artificial implant or the patient's body tissue in a support device. Examples of such support devices are disclosed in U.S. Patent Publication No. 2013/0253645 A1 to Kerr et al. In this publication, a three-dimensional medical support device is disclosed, which preferably is knitted from polymeric yarns, threads, filaments or monofilaments and which can have an elongation of 0-30 % along a horizontal axis and an elongation of 0-10 % along a vertical axis, which is said to give a vertical support against gravity and the force of landing during walking and running and also to give a horizontal support which makes the implant more comfortable and allows a reconstructed breast to appear and feel more natural. WO 2013/012635 discloses a surgical mesh employing silk yarn with different mechanical characteristics in different mesh locations. As recognized in the prior art, support, comfort as well as appearance and feeling of a supported, reconstructed breast are important features of a medical support device for supporting a breast implant or breast tissue; and although efforts have been made to provide a support device with corresponding characteristics, there is still a need for an improved support device, which provides better support for an artificial breast implant or bodily tissue and which also stimulates integration with the existing tissue at the implant site.

### Summary of the Invention

The above-mentioned objects are achieved by the present invention according to the independent claims. Preferred embodiments are set forth in the dependent claims. Insofar as the terms "invention" and/or "embodiment" are used in the following, and/or features are presented as being optional, this should be interpreted in such a way that the only protection sought is that of the invention as claimed. A medical support device according to the present invention is intended to support and optionally also lift natural tissue or artificial material used to reconstruct a female breast structure. In one embodiment, a support device comprises a generally hemispherical, cone- or cup-shaped three-dimensional structure comprising a mesh structure, which mesh structure is an essentially two-dimensional structure, which is formed or shaped into the three-dimensional structure of the medical support device, and which can be made from non-biodegradable or preferably biodegradable polymeric material. In another embodiment, a support device comprises approximately one half of a generally hemispherical, cone- or cup-shaped three-dimensional structure comprising a mesh structure, which mesh structure is an essentially two-dimensional structure, which is formed or shaped into the three-dimensional structure of the medical support device, and which can be made from non-biodegradable or preferably biodegradable polymeric material. According to the invention, the hemispherical or semi-hemispherical three-dimensional structure is further divided into two, three or more portions with different mechanical properties. Typically, a lower or bottom portion (as seen in implanted state) of a three-dimensional mesh structure can be rather inelastic, i.e. having a high modulus of elasticity, and have high strength to provide a lot of support for the implanted breast material, while an upper or top portion (as seen in implanted state) of a three-dimensional mesh structure can be more elastic, i.e. have a low modulus of elasticity, and have less strength to provide more comfort for the person having a reconstructed breast.

### Brief Description of the Drawings

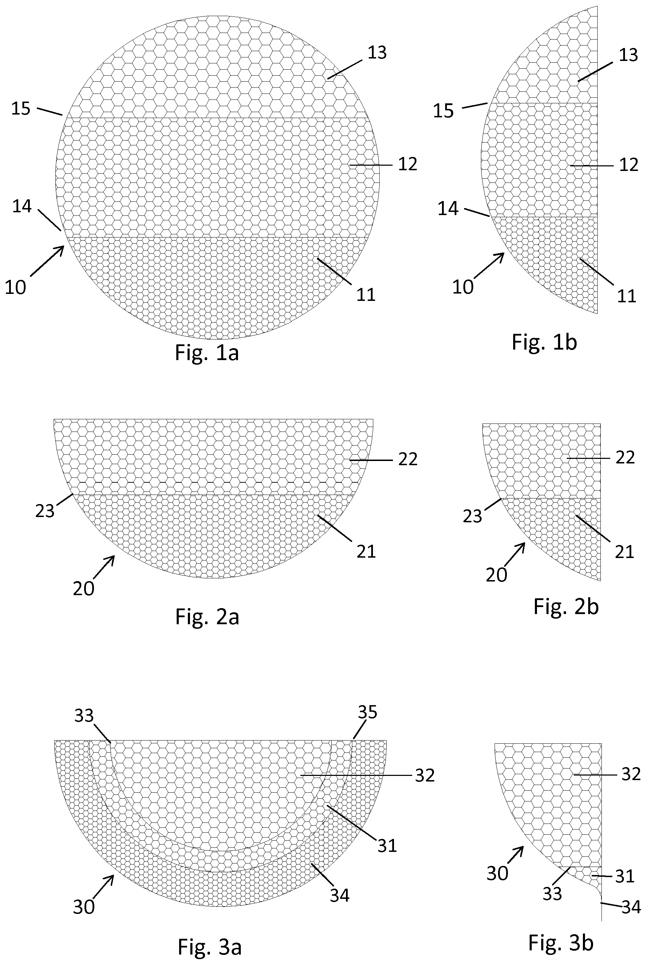

Fig. 1a shows a front view and Fig. 1b shows a side view of a first embodiment of a medical support device according to the present invention, wherein the support device comprises a generally hemispherical mesh structure having three portions with different values of one or more specific mechanical properties.
Fig. 2a shows a front view and Fig. 2b shows a side view of a second embodiment of a medical support device according to the present invention, wherein the support device comprises one half of a generally hemispherical mesh structure having two portions with different values of one or more specific mechanical properties.
Fig. 3a shows a front view and Fig. 3b shows a side view of a third embodiment of a medical support device according to the present invention, wherein the support device comprises one half of a generally hemispherical mesh structure having two portions, including an inner portion and a peripheral outer portion, with different values of one or more specific mechanical properties, and wherein the support device further comprises a flange portion in the shape of an essentially flat mesh structure which is arranged outside of the hemispherical mesh structure.

### Detailed Description of Preferred Embodiments

The present invention relates to a medical support device for supporting a (typically female) breast after a medical breast reconstruction surgery. A breast reconstruction surgery generally involves the re-creation of a breast either by using the patient's own tissue and/or using an artificial medical implant made from, e.g., silicone or saline. For this purpose, the implanted material can be held in place with a three-dimensional, generally hemispherical, cup-shaped or cone-shaped support device, which can be made from degradable or non-degradable mesh material, which mesh material is an essentially two-dimensional structure, which is formed or shaped into the three-dimensional structure of the medical support device. For some medical cases, approximately one half of a generally hemispherical, cup-shaped or cone-shaped support device can be sufficient to provide the desired support for the implanted reconstruction material; and also other sizes and shapes of a three-dimensional mesh structure suitable for supporting a reconstructed breast are within the scope of the present invention.

Support devices of this kind are known in the art; and are, for example, disclosed in the above-referenced patent document. However, the inventor of the present invention has realized that the mechanical requirements regarding, e.g., strength, elasticity and stability, are not uniformly distributed over the surface of a three-dimensional, generally hemispherical support device used for supporting a reconstructed breast. Instead, typically more support is required at a lower part of the support device, whereas an upper part of the support device should be more mechanically compliant to provide the patient with as much comfort as possible and also to give the reconstructed breast a natural appearance. It can be noted that strictly speaking, terms herein like "lower" or "bottom" and "upper" or "top" refer to a medical support device in its implanted state, but the meaning should nevertheless be obvious for the person skilled in the art.

A first example of a medical support device according to the present invention is schematically depicted in Fig. 1a and Fig. 1b, where Fig. 1a shows a front view and Fig. 1b shows a side view of a support device 10 for supporting artificial and/or natural tissue used to reconstruct a female breast. The support device 10 comprises a three-dimensional, generally hemispherical, cup-shaped or cone-shaped structure 10, which preferably is a synthetic mesh structure 10 and even more preferably a biodegradable synthetic mesh structure 10, which, once implanted, degrades in a human body. Resorbable synthetic meshes of this type are, for example, the ones that are commercially available under the trade name TIGR® Matrix Surgical Mesh and are sold by the company Novus Scientific. This mesh - as well as virtually all meshes, synthetic or non-synthetic, degradable or non-degradable, that are available in the market - can with proper modifications described below be used to fulfill the requirements according to the present invention. The shape of the hemispherical structure 10 corresponds to the form of women's brassieres and could, like women's brassieres, be made in different sizes. As can be seen from Fig. 1a and Fig. 1b, the three-dimensional mesh structure 10 is further divided into three portions: a lower or bottom portion 11, an intermediate or middle portion 12, and an upper or top portion 13.

As has been indicated above, the most basic mechanical requirements on the lower portion 11 are to support and hold an implant or implanted tissue in place, which otherwise has a tendency to become dislocated, i.e. typically move downwards due to gravitation. Therefore the lower portion 11 is characterized by having a high modulus of elasticity (being rather inelastic) and/or having a high degree of stiffness, strength and/or stability. For the upper portion 13 the situation is different, since the mechanical requirements regarding elasticity, strength and stability are lower. The upper portion 13 of the mesh structure 10 should instead be comfortable for the person having a reconstructed breast and should also provide the breast with a natural and appealing overall appearance. Hence, the upper portion 13 is characterized by having a comparatively low modulus of elasticity (being rather elastic) and being compliant and/or having a low degree of stiffness, strength and/or stability. The mechanical requirements for the intermediate portion 12 are somewhere in between the mechanical requirements for the lower portion 11 and the upper portion 13; and the intermediate portion 12 is consequently characterized by having moderately high modulus of elasticity and having a moderate compliance and/or having a moderately high degree of stiffness, strength and/or stability. In this embodiment, there are three portions 11, 12 and 13 indicated in Fig. 1a and Fig. 1b. However, there could be only two portions, or more than three portions, such as four, five or more portions, all of them with different mechanical characteristics. Here it can be mentioned that partition line 14, which separates lower portion 11 and intermediate portion 12, and partition line 15, which separates intermediate portion 12 and upper portion 13, are merely introduced as guides for the eye, and have no direct physical interpretation. However, the transition from one portion to a neighbouring portion could be rather abrupt (as in Fig. 1a and Fig. 1b) or could be more gradual or even continuous such that a support device is characterized by having a high modulus of elasticity (or some other mechanical property) at a bottom rim and a low modulus of elasticity (or some other mechanical property) at a top rim, and a gradual and/or continuous transition of modulus of elasticity (or some other mechanical property) therebetween. In the latter case, an intermediate portion can be characterized by not having a fixed mechanical characteristic, but instead be characterized by having a mechanical characteristic that gradually and/or continuously transforms from the characteristics of a neighbouring lower portion to the characteristics of a neighbouring upper portion. The discussion above about number of portions as well as about transition characteristics between portions apply to all embodiments shown and described herein.

A second embodiment of a medical support device according the present invention is disclosed in Fig. 2a and Fig. 2b, where Fig. 2a shows a front view and Fig. 2b shows a side view of a support device 20 for supporting artificial and/or natural tissue used to reconstruct a female breast. The support device 20 comprises a three-dimensional, generally one half of a hemispherical, cup-shaped or cone-shaped structure 20, which preferably is a synthetic mesh structure 20 and even more preferably a biodegradable synthetic mesh structure 20, which, once implanted, degrades in a human body. As can be seen from Fig. 2a and Fig. 2b, the three-dimensional mesh structure 20 is further divided into two portions: a lower portion 21 and an upper portion 22, as indicated and visualized by partition line 23. In accordance with the teachings discussed in conjunction with the embodiment shown in Fig. 1a and Fig. 1b, the lower portion 21 is characterized by having a high modulus of elasticity (being rather inelastic) and/or having a high degree of stiffness, strength and/or stability, while the upper portion 22 is characterized by having a comparatively low modulus of elasticity (being rather elastic) and being compliant and/or having a low degree of stiffness, strength and/or stability.

A somewhat different division into portions with different mechanical characteristics is disclosed in a third embodiment of a medical support device according the present invention. This embodiment is presented in Fig. 3a and Fig. 3b, where Fig. 3a shows a front view and Fig. 3b shows a side view of a support device 30 for supporting artificial and/or natural tissue used to reconstruct a female breast. The support device 30 comprises a three-dimensional, generally one half of a hemispherical, cup-shaped or cone-shaped structure 30, which preferably is a synthetic mesh structure 30 and even more preferably a biodegradable synthetic mesh structure 30, which, once implanted, degrades in a human body. As can be seen from Fig. 3a and Fig. 3b, the three-dimensional mesh structure 30 is further divided into two portions: an outer portion 31 and an inner or central portion 32, as indicated and visualized by partition line 33. The outer portion 31 is arranged to provide support and hold an implant or implanted tissue in place, and is therefore characterized by having a high modulus of elasticity (being rather inelastic) and/or having a high degree of stiffness, strength and/or stability. The inner central portion 32 is arranged to provide comfort for the person having a reconstructed breast and should also provide the breast with a natural appearance, and is therefore characterized by having a comparatively low modulus of elasticity (being rather elastic) and being compliant and/or having a low degree of stiffness, strength and/or stability. It can in particular be noted from Fig. 3b that the inner portion 32 and the outer portion 31 are parts of the three-dimensional part of support device 30, and that outside of the three-dimensional mesh structure 30 there is another mesh structure 34 provided. The mesh structure 34 is arranged as a peripheral flange structure 34 and is arranged outside of the outer portion 31, and also outside of the three-dimensional part of medical support device 30. All embodiments envisaged herein could be provided with a similar flat flange portion. In particular, the configuration with an outer, peripheral rim or flange portion and one or more inner portions can easily be transferred to a full hemispherical mesh structure, which then comprises an inner portion with a first set of mechanical characteristics and one circumferential outer portion, which encloses the inner portion, with a second set of mechanical characteristics; or two or more outer portions with individual mechanical characteristics, e.g. a lower flange portion with a high modulus of elasticity and an upper flange portion with a low modulus of elasticity.
Different techniques can be utilized to produce a medical support device according to the present invention. In an embodiment of the present invention, in knitting or weaving techniques, knitting or weaving patterns can be changed from one portion to another, e.g. by varying pore sizes or number of fibers per surface area or include more fibers with higher modulus of elasticity. It is also possible to sew or otherwise join together different mesh portions having different mechanical properties, or even for some portions to arrange two or more layers of mesh on top of each other. To create the three-dimensional shape, i.e. to produce the hemispherical cup-shape, essentially two-dimensional meshes are commonly heat treated. This process, which in the art also is referred to as annealing, means that mesh material is exposed to heat, typically by placing the mesh material in mould which is heated to a specific temperature. With suitable choice of material, e.g. polymeric material, the mesh material and thereby the medical support device will adopt the shape of the mould. To produce portions with different mechanical characteristics, certain areas of the mesh material can be exposed to more heat, i.e. higher temperature or longer dwell time in the mould. For example, with reference back to the third embodiment described in conjunction with Fig. 3a and Fig. 3b, higher temperature and/or longer dwell time can be provided for the outer portion 31 than for the inner central portion 32, and even higher temperature and/or long dwell time can be provided for flange portion 34, to thereby create a medical support device having a high modulus of elasticity at its rim and a low modulus of elasticity in its central portion. The temperature over the mould can be varied, for example such that a heat gradient profile is maintained which gives the mesh material a sharp, gradual or continuous transition from one part of the mesh structure to another part of the mesh structure. Many mechanical properties of a mesh structure can be modified, including, but not limited to, extension, modulus of elasticity, compliance, stiffness, yield strength, tensile strength, or elongation.

As indicated above, one mechanical property that can characterize a mesh structure according to the invention is extension. Extension is preferably measured using the ASTMD D3787-1 guideline for ball burst strength, with some preset parameters, such as a preload of 0.1 N and a test speed of 75 mm/min. As a non-limiting example, the extension (travel distance of the ball) measured at a load of 50 N, for a bottom or lower portion, which is characterized by having a high modulus of elasticity, a high degree of stiffness, strength and/or stability, can be in the interval of 2 mm to 10 mm, while for an upper or top portion, which is characterized by having a comparatively low modulus of elasticity and being mechanically compliant and/or having a low degree of stiffness, strength and/or stability, the extension can be in the interval of 5 mm to 15 mm, with the provision that the extension for the bottom portion is lower than the extension for the top portion of each individual support device. Intermediate portions of a mesh structure can have values between these two intervals.
As another non-limiting example, the extension measured at a load of 50 N, for an outer portion 31, which is characterized by having a high modulus of elasticity, a high degree of stiffness, strength and/or stability, can be in the interval of 2 mm to 10 mm, while for an inner portion 32, which is characterized by having a comparatively low modulus of elasticity and being mechanically compliant and/or having a low degree of stiffness, strength and/or stability, the extension can be in the interval of 5 mm to 15 mm, with the provision that the extension for the outer portion is lower than the extension for the inner portion of each individual support device.

Although the present invention has been described with reference to specific embodiments, also shown in the appended drawings, it will be apparent to those skilled in the art that many variations and modifications can be done within the scope of the invention as described in the specification and defined with reference to the claims below.

## Claims

1. A medical support device, comprising a three-dimensional mesh structure (10; 20; 30) for supporting a breast implant and/or tissue, wherein the three-dimensional mesh structure (10; 20; 30) is made from an essentially two-dimensional mesh material formed and shaped into said three-dimensional structure, and wherein the three-dimensional mesh structure (10; 20; 30) comprises at least a first portion (11; 21; 31) of the two-dimensional mesh material, which has a first value of a specific mechanical property, and a second portion (12; 22; 32) of the two-dimensional mesh material, which has a second value of said specific mechanical property, which second value of said specific mechanical property is different from the first value of said mechanical property, and
wherein said mechanical property can be anyone of extension, modulus of elasticity, compliance, stiffness, yield strength, tensile strength, or elongation, and **characterised in that** said first portion (11; 21; 31) and said second portion (12; 22; 32) differ from each other by at least one of the following: different pore size or number of fibers per surface area or number of fibers with higher modulus of elasticity and that the two-dimensional mesh material is a knitted or woven material.

2. A medical support device according to claim 1, wherein the three-dimensional mesh structure (10; 20; 30) has a curvature corresponding to a generally hemispherical shape, or a part thereof.

3. A medical support device according to any one of claims 1 to 2, wherein the first portion of the three-dimensional mesh structure (20) is configured to be a bottom portion (21) and the second portion is configured to be a top portion (22) when the medical support device is in an implanted state.

4. A medical support device according to any one of claims 1 to 2, wherein the first portion of the three-dimensional mesh structure (30) is an outer portion (31), and the second portion is an inner portion (32).

5. A medical support device according to any one of claims 1 to 2, wherein the three-dimensional mesh structure (10) comprises a third portion, which has a third value of said specific mechanical property, which third value of said specific mechanical property is different from both the first value and the second value of said specific mechanical property.

6. A medical support device according to claim 5, wherein the first portion of the three-dimensional mesh structure (10) is configured to be a bottom portion (11), the second portion is configured to be an intermediate portion (12) and the third portion is configured to be a top portion (13) when the medical support device is in an implanted state.

7. A medical support device according to any one of claims 1 to 6, wherein the medical support device further has an outer flange portion (34), which comprises an essentially flat mesh structure (34) and is arranged outside of the three-dimensional mesh structure.

## Patentansprüche

1. Medizinische Trägervorrichtung, umfassend eine dreidimensionale Maschenstruktur (10; 20; 30) zum Tragen eines Brustimplantats und/oder -gewebes, wobei die dreidimensionale Maschenstruktur (10; 20; 30) aus einem im Wesentlichen zweidimensionalen Maschenmaterial hergestellt ist, das zu der dreidimensionalen Struktur ausgebildet und geformt ist, und wobei die dreidimensionale Maschenstruktur (10; 20; 30) mindestens einen einen ersten Wert einer spezifischen mechanischen Eigenschaft aufweisenden ersten Teil (11; 21; 31) des zweidimensionalen Maschenmaterials und einen einen zweiten Wert der spezifischen mechanischen Eigenschaft aufweisenden zweiten Teil (12; 22; 32) des zweidimensionalen Maschenmaterials umfasst, wobei sich der zweite Wert der spezifischen mechanischen Eigenschaft vom ersten Wert der mechanischen Eigenschaft unterscheidet, und
wobei die mechanische Eigenschaft eine beliebige aus Ausdehnung, Elastizitätsmodul, Nachgiebigkeit, Steifigkeit, Streckfestigkeit, Zugfestigkeit oder Zugdehnung sein kann
und **dadurch gekennzeichnet, dass**
sich der erste Teil (11; 21; 31) und der zweite Teil (12; 22; 32) durch mindestens eines aus Folgendem voneinander unterscheiden: unterschiedliche Porengröße oder Anzahl von Fasern pro Oberflächenbereich oder Anzahl von Fasern mit höherem Elastizitätsmodul, und dass das zweidimensionale Maschenmaterial ein geknüpftes oder gewebtes Material ist.

2. Medizinische Trägervorrichtung nach Anspruch 1, wobei die dreidimensionale Maschenstruktur (10; 20; 30) eine Wölbung aufweist, die einer im Allgemeinen hemisphärischen Gestalt oder einem Teil davon entspricht.

3. Medizinische Trägervorrichtung nach einem der Ansprüche 1 bis 2, wobei der erste Teil der dreidimensionalen Maschenstruktur (20) als ein unterer Teil (21) konfiguriert ist und der zweite Teil als ein oberer Teil (22) konfiguriert ist, wenn die medizinische Trägervorrichtung in einem implantierten Zustand vorliegt.

4. Medizinische Trägervorrichtung nach einem der Ansprüche 1 bis 2, wobei der erste Teil der dreidimensionalen Maschenstruktur (30) ein Außenteil (31) ist und der zweite Teil ein Innenteil (32) ist.

5. Medizinische Trägervorrichtung nach einem der Ansprüche 1 bis 2, wobei die dreidimensionale Maschenstruktur (10) einen dritten Teil umfasst, der einen dritten Wert der spezifischen mechanischen Eigenschaft aufweist, wobei sich der dritte Wert der spezifischen mechanischen Eigenschaft sowohl vom ersten Wert als auch vom zweiten Wert der spezifischen mechanischen Eigenschaft unterscheidet.

6. Medizinische Trägervorrichtung nach Anspruch 5, wobei der erste Teil der dreidimensionalen Maschenstruktur (10) als ein unterer Teil (11) konfiguriert ist, der zweite Teil als ein Zwischenteil (12) konfiguriert ist und der dritte Teil als ein oberer Teil (13) konfiguriert ist, wenn die medizinische Trägervorrichtung in einem implantierten Zustand vorliegt.

7. Medizinische Trägervorrichtung nach einem der Ansprüche 1 bis 6, wobei die medizinische Trägervorrichtung ferner einen äußeren Bundteil (34) aufweist, der eine im Wesentlichen flache Maschenstruktur (34) umfasst und außerhalb der dreidimensionalen Maschenstruktur angeordnet ist.

## Revendications

1. Dispositif médical de support, comprenant une structure maillée tridimensionnelle (10 ; 20 ; 30) pour supporter un implant et/ou tissu mammaire, dans lequel la structure maillée tridimensionnelle (10 ; 20 ; 30) est constituée d'un matériau maillé essentiellement bidimensionnel formé et façonné en ladite structure tridimensionnelle, et dans lequel la structure maillée tridimensionnelle (10 ; 20 ; 30) comprend au moins une première portion (11 ; 21 ; 31) du matériau maillé bidimensionnel, laquelle présente une première valeur d'une propriété mécanique spécifique, et une deuxième portion (12 ; 22 ; 32) du matériau maillé bidimensionnel, laquelle présente une deuxième valeur de ladite propriété mécanique spécifique, laquelle deuxième valeur de ladite propriété mécanique spécifique est différente de la première valeur de ladite propriété mécanique, et
dans lequel ladite propriété mécanique peut être une quelconque parmi une extension, un module d'élasticité, une compliance, une rigidité, une limite d'élasticité, une résistance à la traction, ou un allongement, et **caractérisé en ce que**
ladite première portion (11 ; 21 ; 31) et ladite deuxième portion (12 ; 22 ; 32) sont différentes l'une de l'autre par au moins un des éléments suivants : une taille de pores ou un nombre de fibres par surface spécifique ou un nombre de fibres avec un module d'élasticité supérieur différents et que le matériau maillé bidimensionnel est un matériau tricoté ou tissé.

2. Dispositif médical de support selon la revendication 1, dans lequel la structure maillée tridimensionnelle (10 ; 20 ; 30) présente une courbure correspondant à une forme généralement hémisphérique, ou une partie de celle-ci.

3. Dispositif médical de support selon l'une quelconque des revendications 1 à 2, dans lequel la première portion de la structure maillée tridimensionnelle (20) est conçue pour être une portion inférieure (21) et la deuxième portion est conçue pour être une portion supérieure (22) lorsque le dispositif médical de support est dans un état implanté.

4. Dispositif médical de support selon l'une quelconque des revendications 1 à 2, dans lequel la première portion de la structure maillée tridimensionnelle (30) est une portion externe (31), et la deuxième portion est une portion interne (32) .

5. Dispositif médical de support selon l'une quelconque des revendications 1 à 2, dans lequel la structure maillée tridimensionnelle (10) comprend une troisième portion, laquelle présente une troisième valeur de ladite propriété mécanique spécifique, laquelle troisième valeur de ladite propriété mécanique spécifique est différente à la fois de la première valeur et de la deuxième valeur de ladite propriété mécanique spécifique.

6. Dispositif médical de support selon la revendication 5, dans lequel la première portion de la structure maillée tridimensionnelle (10) est conçue pour être une portion inférieure (11), la deuxième portion est conçue pour être une portion intermédiaire (12) et la troisième portion est conçue pour être une portion supérieure (13) lorsque le dispositif médical de support est dans un état implanté.

7. Dispositif médical de support selon l'une quelconque des revendications 1 à 6, dans lequel le dispositif médical de support présente en outre une portion de rebord externe (34), qui comprend une structure maillée essentiellement plate (34) et est disposée à l'extérieur de la structure maillée tridimensionnelle.
